# EUROPEAN PATENT APPLICATION

(11) **EP 4 427 697 A1**
(43) Date of publication of application: **11.09.2024**
(21) Application number: 23305312.3
(22) Date of filing: 08.03.2023
(51) Int. Cl.: A61B 34/30

(54) **HYBRID CONTROL OF A SURGICAL SYSTEM**

(71) Applicant: MinMaxMedical, 38400 Saint-Martin-d'Hères (FR)
(72) Inventor: QUINTIN, Jérémy, 38400 SAINT-MARTIN-D'HÈRES (FR); FONTAINE, Sylvain, 38400 SAINT-MARTIN-D'HÈRES (FR); FOURNIER, Elie, 38400 SAINT-MARTIN-D'HÈRES (FR); LONJARET, Thomas, 38400 SAINT-MARTIN-D'HÈRES (FR); MARCOL, Cyrill, 38400 SAINT-MARTIN-D'HÈRES (FR); LAVALLEE, Stéphane, 38400 SAINT-MARTIN-D'HÈRES (FR)
(74) Representative: Regimbeau

(57) **Abstract**

The present invention concerns a surgical system (100) configured to treat a region of interest of an anatomical structure, the surgical system (100) comprising
• at least one robotic arm (110) coupled with a surgical tool (121) configured to treat the region of interest, the robotic arm (110) comprising several links (111), two adjacent links (111) being linked to one another by one motorized joint (112), the robotic arm (110) comprising at least a first part (113) with at least three degrees of freedom and a second part (114) with at least six degrees of freedom, an end of the first part (113) forming a base (115) of the second part (114) and the first part (113) comprising a selective compliance assembly robot arm,
• at least one control device (122) configured to receive commands from a user of the system (100), and
• at least one data processor (103),
the surgical system (100) being operable, at least, according to a hand-guiding mode, the data processor (103) being configured to, as long as the hand-guiding mode is enabled:
• determine a current dynamic condition of the robotic arm (110),
• receive at least one user command from the control device (122),
• determine, based on the received user command(s) and on the current dynamic condition of the robotic arm (110), an intended displacement of the surgical tool (121),
• determine at least one optimal trajectory of the robotic arm (110) permitting to obtain the determined intended displacement of the surgical tool (121), while satisfying at least one mathematical function which defines authorized trajectories of the robotic arm (110),
• compute and send at least one instruction(s) to at least one of the motorized joint(s) (112) of the robotic arm (110), the execution of the at least one instruction(s) (i4) resulting in a displacement of the robotic arm (110) according to the optimal trajectory,
wherein when several optimal trajectories of the robotic arm (110) are determined, the data processor (103) is configured to select one of these optimal trajectories so that the displacement of the robotic arm (110) implies the smallest number of movements of the motorized joint(s) of the first part (113) possible.

## Description

The present invention concerns the domain of surgical systems, and especially, it concerns a surgical system adapted to be used for orthopedic surgeries.

During the last few decades, the field of surgical systems has significantly grown, and especially surgical systems which are adapted to be used in computer-assisted medical intervention (CAMI), as referred to in the publication of S. Lavallée & P. Cinquin: "Computer assisted medical interventions" In K.H. Hohne, editor, NATO ARW, 30 Imaging in Medicine, Vol F60, 301-312, Berlin, June 1990. Springer-Verlag. Such systems aim to help surgeons in performing securer operations while also improving the accuracy and reproducibility of said operations. The use of those surgical systems also aims at improving the precision of surgical movements, lower invasiveness and in reducing the number and the seriousness of errors that can otherwise occur during those operations.

Robotic aid to clinicians for execution of optimal surgery was introduced in the early 1980's in neurosurgery application. Since the 1990's, several assistive technologies combining part or all of 2D/3D imaging, navigation and robotics were developed with the primary focus of improving accuracy of surgical procedures, in view of improving clinical and functional outcomes for the patients.

A first generation of robots were developed as passive-robots. These passive-robots can for example consist of optical localizer or motorless encoded arms and they are particularly well-suited for navigation, but their use is difficult for executing complex surgical strategies. However, these passive-robots can be useful for performing simple operation such as ones wherein the movements needed are all about one single axis. For instance, the robot Cirq^{®} developed by BrainLab is one of those passive robots.

A second generation of robots were developed as active robots. These active robots are designed to perform at least part of an intervention on their own from a planned procedure, i.e., without any real-time guidance from the surgeon, nor from any other operator. For instance, Robodoc^{®} is an active robot developed by Integrated Surgical Systems, commercialized at the end of the 90's and adapted to perform part of hip replacements surgeries. Such active robots are generally accurate but raise safety and ergonomics issues.

Nowadays, many medical device suppliers are developing collaborative robots, i.e., robots with which the operators are able to cooperate. These collaborative robots are equipped with an input device through which the user of the system can ask for a displacement of the robot, and the system is then adapted to treat the received input and to displace the robot literally as asked by the user or to forbid the displacement asked, i.e. taking into account both the demand of the user and, for instance, safety parameter. One major drawback of those collaborative robots is that they are particularly adapted to be used for one dedicated kind of surgery, thus forcing the users to buy several robots if they want to be able to perform different surgeries.

One solution to increase the number of surgeries which can be realized with the help of one unique collaborative robot is to increase the number of degrees of freedom of such collaborative robots. One major issue of this increasing is that the robots become more and more complicated to drive for the user, especially when said collaborative robots are equipped with a unique input device.

Thus, there remains a need to provide a collaborative surgical system which can be used for several kind of surgeries, while being easy to drive for its user and safe both for the patient to be treated and for the surgeon and all the caregivers which can be in the surrounding of said system during surgical interventions.

The present invention falls in this context and aims at providing such a collaborative system.

An object of the present invention thus relates to a surgical system configured to treat a region of interest of an anatomical structure, the surgical system comprising
- at least one robotic arm coupled with a surgical tool configured to treat the region of interest, the robotic arm comprising several links, two adjacent links being linked to one another by one motorized joint, the robotic arm comprising at least a first part with at least three degrees of freedom and a second part with at least six degrees of freedom, an end of the first part forming a base of the second part and the first part comprising a selective compliance assembly robot arm,
- at least one control device configured to receive commands from a user of the system, and
- at least one data processor,
the surgical system being operable, at least, according to a hand-guiding mode, the data processor being configured to, as long as the hand-guiding mode is enabled:
- determine a current dynamic condition of the robotic arm,
- receive at least one user command from the control device,
- determine, based on the received user command(s) and on the current dynamic condition of the robotic arm, an intended displacement of the surgical tool,
- determine at least one optimal trajectory of the robotic arm permitting to obtain the determined intended displacement of the surgical tool, while satisfying at least one mathematical function which defines authorized trajectories of the robotic arm,
- compute and send at least one instruction(s) to at least one of the motorized joint(s) of the robotic arm, the execution of the at least one instruction(s) resulting in a displacement of the robotic arm according to the optimal trajectory,
wherein when several optimal trajectories of the robotic arm are determined, the data processor is configured to select one of these optimal trajectories so that the displacement of the robotic arm implies the smallest number of movements of the motorized joint(s) of the first part possible.

The invention can be complemented by the following features taken alone or in combination:
The control device comprises at least one force and/or torque sensor and the control device is configured to, as long as the hand-guiding mode is enabled:
- measure forces and/or torques applied by the user on the control device,
- determine the at least one user command based on the measured forces and/or torques,
- send the user command to the data processor.

The data processor is configured to detect and/or measure a force and/or torque applied on at least one of the motorized joints of the second part of the robotic arm, and the data processor is further configured to, as long as one of the hand-guiding mode is enabled:
- determine, based on the detected and/or measured force and/or torque applied on the concerned motorized joint(s), a configuration of the concerned motorized joint(s) intended by the user,
- select the optimal trajectory of the robotic arm so that the displacement of the concerned motorized joint(s) matches the intended configuration of said motorized joint(s).

The surgical system is associated with at least one tracking system configured to determine, at a predefined frequency, relative pose of the surgical tool with respect to the anatomical structure and with respect to the region of interest and to send a corresponding information to the data processor.

To determine the pose of the surgical tool with respect to the region of interest, the tracking system can for instance be configured to first determine the relative pose of the surgical tool with respect to the anatomical structure. Then, as the pose of the region of interest with respect to the anatomical structure is known, the tracking system is configured to determine the relative pose of the surgical tool with respect to the region of interest, based on the determined pose of the surgical tool with respectto the anatomical structure and on the known pose of the region of interest with respect to the anatomical structure. Alternatively, the tracking system can be configured to determine the relative pose of the surgical tool with respect to the anatomical structure and to send a corresponding information to the data processor, the data processor then being configured to determine the pose of the surgical tool with respect to the region of interest.

The tracking system can for instance be an optical tracking system, an electromagnetic tracking system, an inertial tracking system or a combination thereof.

The surgical system comprises at least one storage unit storing a surgical plan defining, at least one initial pose of the surgical tool allowing a user of the system to start treating the region of interest, wherein the surgical system is operable according to a pre-alignment mode and wherein the data processor is configured to, as long as the pre-alignment mode is enabled:
- determine a current configuration of the robotic arm,
- determine, based on the stored surgical plan and on the determined current configuration of the robotic arm, a displacement of the surgical tool needed to reach its initial pose,
- determine at least one optimal trajectory of the robotic arm permitting to obtain the determined displacement of the surgical tool, while satisfying at least one mathematical function which defines authorized trajectories of the robotic arm,
- compute and send at least one instruction(s) to at least one of the motorized joint(s) of the robotic arm, the execution of the at least one instruction(s) resulting in a displacement of the robotic arm according to the optimal trajectory,
wherein when several optimal trajectories of the robotic arm are determined, the data processor is configured to select one of these optimal trajectories so that the displacement of the robotic arm implies the smallest number of displacements of the motorized joint(s) of the first part possible.

The initial pose can be registered as an absolute pose in a defined reference system. For instance, the initial pose can be registered as an absolute pose in a reference system associated with the robotic arm. Alternatively, the initial pose can be registered as a relative pose of the surgical tool with respect to the region of interest. According to this alternative, the data processor is configured to, as long as the pre-alignment mode is enabled, determine the displacement of the surgical tool needed to reach its initial pose based on the determined current configuration of the robotic arm, on the stored surgical plan and on the determined relative pose of the surgical tool with respect to the region of interest.

The surgical system is operable in an interactive mode, the data processor being configured to, as long as the interactive mode is enabled:
- determine the current dynamic condition of the robotic arm,
- receive at least one user command from the control device,
- determine, based on the received user command(s) and on the current dynamic condition of the robotic arm, the intended displacement of the surgical tool,
- receive the information related to the determined relative pose of the surgical tool with respect to the region of interest from the tracking system,
- determine a predictive pose of the surgical tool with respect to the anatomical structure based on the determined intended displacement of the surgical tool and on the received information related to the determined relative pose of the surgical tool with respect to the region of interest.

If the predictive pose of the surgical tool with respect to the anatomical structure matches a stored pose of the region of interest, the data processor is further configured to:
- determine at least one optimal trajectory of the robotic arm permitting to obtain the determined intended displacement of the surgical tool, while satisfying at least one mathematical function which defines authorized trajectories of the robotic arm,
- compute and send at least one instruction(s) to at least one of the motorized joint(s) of the robotic arm, the execution of the at least one instruction(s) resulting in a displacement of the robotic arm according to the optimal trajectory.

The surgical system is operable in an interactive mode, and, as long as the interactive mode is enabled, the motorized joints of the first part of the robotic arm are locked.

Satisfying the at least one mathematical function ensures to avoid one or several of the following:
- Singularities,
- Collisions with objects which are distinct from the robotic arm, and which are in the surroundings of the surgical system,
- Collisions between distinct links of the robotic arm,
- Collisions between the surgical tool and the robotic arm,
- Collisions between the robotic arm and a robot station.

Thus, when it is referred to "at least one mathematical function" in the present document, it is referred to at least one mathematical function chosen among identical limited lists of mathematical functions.

The surgical tool is deactivated as long as the hand-guiding mode or the pre-alignment mode is enabled.

The surgical system comprises exactly one control device.

The first part of the robotic arm comprises three motorized joints, two of which permitting to displace the base of the second part within a plane and a third one permitting to displace the base of the second part along an axis perpendicular to said plane, and the second part of the robotic arm comprises at least seven motorized joints.

The surgical tool is a cutting tool or a cutting guide.

The present invention also concerns a method for controlling a surgical system operable in a hand-guiding mode, the surgical system comprising
- at least one robotic arm coupled with a surgical tool, the robotic arm comprising several links, two adjacent links being linked to one another by one motorized joint, the robotic arm comprising at least a first part with at least three degrees of freedom and a second part with at least six degrees of freedom, an end of the first part forming a base of the second part and the first part comprising a selective compliance assembly robot arm,
the method comprising, as long as the hand-guiding mode is enabled, the steps of
- determining a current dynamic condition of the robotic arm,
- receiving at least one user command from the control device,
- determining, based on the received user command(s) and on the current dynamic condition of the robotic arm, an intended displacement of the surgical tool,
- determining at least one optimal trajectory of the robotic arm permitting to obtain the determined displacement of the surgical tool, while satisfying at least one mathematical function which defines authorized trajectories of the robotic arm,
- computing and sending at least one instruction(s) to at least one of the motorized joint(s) of the robotic arm, the execution of the at least one instruction(s) resulting in a displacement of the robotic arm according to the optimal trajectory,
wherein when several optimal trajectories of the robotic arm are determined, the method comprises an additional step of selecting one of these optimal trajectories so that the displacement of the robotic arm implies the smallest number of movements of the motorized joint(s) of the first part possible.

The present invention further concerns a method for controlling a surgical system configured to treat a region of interest of an anatomical structure, the surgical system being operable in an interactive mode, the surgical system comprising
- at least one robotic arm coupled with a surgical tool, the robotic arm comprising several links, two adjacent links being linked to one another by one motorized joint, the robotic arm comprising at least a first part with at least three degrees of freedom and a second part with at least six degrees of freedom, an end of the first part forming a base of the second part and the first part comprising a selective compliance assembly robot arm,
- at least one tracking system configured to determine, at a predefined frequency, relative pose of the surgical tool with respect to the anatomical structure and with respect to the region of interest,
the method comprising, as long as the interactive mode is enabled, the steps of
- determining the current dynamic condition of the robotic arm,
- receiving at least one user command from the control device,
- determining, based on the received user command(s) and on the current dynamic condition of the robotic arm, the intended displacement of the surgical tool,
- receiving an information related to the determined relative pose of the surgical tool with respect to the region of interest from the tracking system,
- determining a predictive pose of the surgical tool with respect to the anatomical structure based on the determined intended displacement of the surgical tool and on the received information related to the determined relative pose of the surgical tool with respect to the region of interest.

If the predictive pose of the surgical tool with respect to the anatomical structure matches a stored pose of the region of interest, the method comprises the additional steps of:
- determining at least one optimal trajectory of the robotic arm permitting to obtain the determined intended displacement of the surgical tool, while satisfying at least one mathematical function which defines authorized trajectories of the robotic arm,
- computing and sending at least one instruction(s) to at least one of the motorized joint(s) of the robotic arm, the execution of the at least one instruction(s) resulting in a displacement of the robotic arm according to the optimal trajectory.

Further details and advantages of the invention will appear in the following description. Embodiments of the invention will be described with reference to the drawings, in which:
- Figure 1 illustrates, in a perspective view, a surgical system according to an embodiment of the invention
- Figures 2 to 4 are diagrams schematically illustrating, respectively, the surgical system of the invention operated according to a hand-guiding mode, to a pre-alignment mode, and to an interactive mode,
- Figures 5 to 8 illustrate successive steps of a surgical plan for preparing a knee to receive a graft in a knee ligamentoplasty;
- Figures 9 to 15 illustrate successive steps of a surgical plan for preparing a knee to receive a knee prosthesis in a total knee arthroplasty.

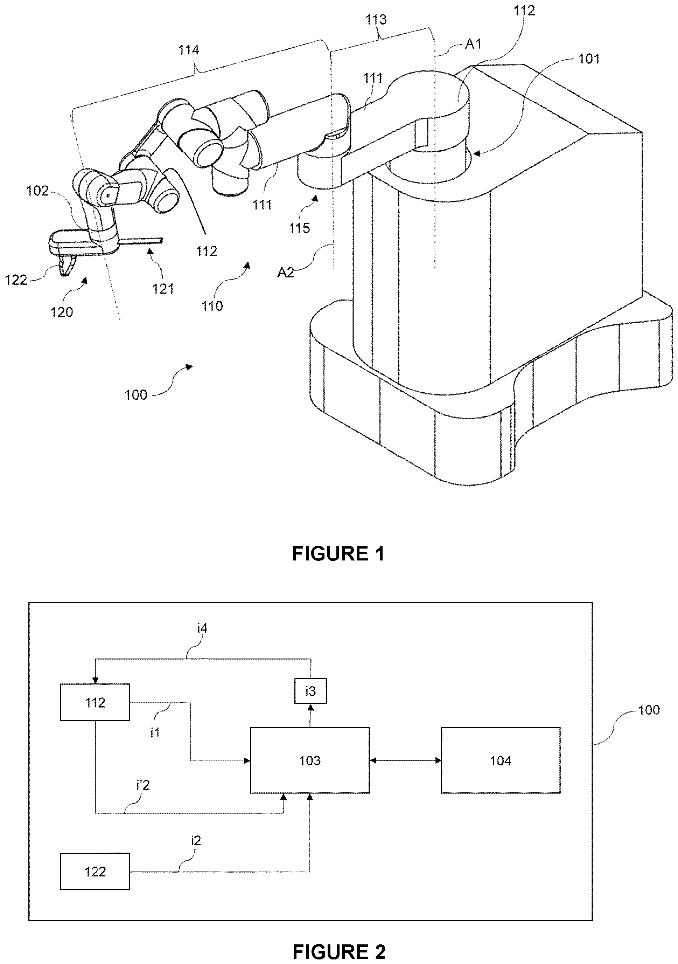

Figure 1 illustrates, in a perspective view, a surgical system 100 according to an embodiment of the invention. In the present specification, the surgical system 100 is also referred to as "the system 100".

The system 100 comprises at least one robotic arm 110 extending between a first end 101 and a flange 102 to which an end-effector 120 is attached. According to the illustrated embodiment, the first end 101 is formed as a fixed cart, but the robotic arm 110 could alternatively be fixed to a wheeled cart or to a surgical table for instance, within the scope of the invention. The system 100 can also comprise a robot station - not shown here - on which can for instance be attached a display.

The end-effector 120 comprises, at least, a surgical tool 121 adapted to treat a region of interest of an anatomical structure of a patient and a control device 122. The control device 122 could be attached to any other location of the robotic arm within the scope of the invention. The robotic arm 110 is coupled with the surgical tool 121. According to the illustrated embodiment, the surgical tool is here formed as an oscillating saw. Obviously, the surgical tool could be formed as any other kind of cutting tool, for instance a burr, a drill or a screwdriver, without departing from the scope of the invention. Alternatively, the surgical tool could also be a cutting guide adapted to guide a cutting tool. Advantageously, the end-effector 120 can be detachably attached to the flange 102 of the robotic arm 110, so that it can be changed between two successive surgeries, or during the course of one surgery if needed.

The control device 122 is configured to receive commands from a user of the system 100. As detailed below, the user of the system 100 can use the control device 122 to transmit a displacement he/she intends to apply to the surgical tool 121 to a data processor of the system 100. According to the illustrated embodiment, the control device 122 is formed as a handle coupled to a force and/or torque sensor. The control device 122, and especially the associated force and/or torque sensor, is thus configured to measure forces and/or torques applied to it by the user of the system and to determine, based on said measured forces and/or torques, a corresponding user command. As detailed below, the control device 122 is then adapted to send a corresponding information to a data processor of the system 100.

Alternatively, the control device 122 could be formed as a joystick configured to measure displacements applied to it, or as any other known input device adapted to receive commands from the user of the system 100. According to the illustrated embodiment, the system 100 comprises exactly one control device 122. Optionally, the system could comprise two or more control devices, for instance for safety reasons as if one of the control devices stops working, the user can pull the surgical tool out of the patient with the other one.

The system 100 further comprises at least one data processor 103 and at least one storage unit 104 - both schematically illustrated on figure 2. The data processor 103 is in communication with the control device 122 and with the storage unit 104. The communication can be wired or wireless within the scope of the invention. The data processor 103 and the storage unit 104 are depicted as two separate components, but they could be formed as a single unit without departing from the scope of the invention. The data processor 103 can, for instance, comprise one or more microprocessor, one or more calculators, one or more computers, one or more computer programs and/or one or more graphic processing unit. The computer program(s) comprise code instructions to compute the needed instructions to be sent to motorized joints of the robotic arm, as described below. In addition, the data processor may include other devices and circuitry for performing the functions described herein such as, for example, a hard drive, input/output circuitry, and the like. The input/output circuitry can be adapted to treat digital and/or analog signals. The storage unit 104 can comprise one or more random access memory (RAM) and/or one or more read-only memory (ROM) and/or one or more non-transitory computer readable medium.

The robotic arm 110 comprises several links 111 linked to one another by motorized joints 112. The robotic arm 110 comprises a first part 113 which presents at least three degrees of freedom and a second part 114 which presents at least six degrees of freedom. Advantageously, the second part 114 of the robotic arm can comprise at least seven degrees of freedom, thus providing at least one redundant degree of freedom. Therefore, the robotic arm 110 comprises at least nine degrees of freedom, advantageously ten degrees of freedom.

As shown, an end of the first part 113 forms a base 115 of the second part 114, and this second part 114 ends at the flange 102 described above. The first part 113 of the robotic arm 110 is particularly realized as a SCARA (Selective Compliance Assembly Robot Arm) and thus comprises two links linked to one another by two motorized joints. As well known in the art, a SCARA comprises at least one translational degree of freedom along a first axis A1, and two axes of rotation - here referenced as A1 and A2 - parallel to one another. The first part 113 of the robotic arm 110 thus permits to displace the second part 114 of the robotic arm 110, and especially the base of this second part 114, within a defined plane, perpendicular to the axes of rotation A1, A2 of the motorized joints of said first part 113. As mentioned, this SCARA is also displaceable along a vertical axis coincident with the axis of rotation A1 of one of its motorized joints, especially coincident the axis of rotation arranged the closer to the first end 101 of the robotic arm 110.

The second part 114 of the robotic arm comprises a wrist to which the surgical tool 121 is attached, the wrist comprising at least three motorized joints 112, among which two roll-joints and one pitch-joint.

Optionally, each motorized joint 112 of the robotic arm 110 can comprise at least one encoder - not illustrated here - configured to determine, at any time, a current configuration of the concerned motorized joint. The data processor 103 is also in communication - whether wired or wireless - with the motorized joints and especially with the motors of said motorized joints, as well as with the encoders of said motorized joints. Thus, the data processor can retrieve, at any time, from those encoders a configuration of each motorized joint and to determine, based on successive retrieved configurations of the motorized joints and on known models of said robotic arm, a current dynamic condition of the robotic arm. The "current dynamic condition of the robotic arm" is, in the present document, defined as a condition of the robotic arm which includes information related to its pose (i.e. the pose of its motorized joints) and/or its acceleration and/or its speed and/or the pose of the surgical tool held by such robotic arm and/or an acceleration of the surgical tool and/or a speed of the surgical tool. Obviously, here is only described one example of how to determine said dynamic condition of the robotic arm, but other known technics and/or device(s) could be used within the scope of the invention. For instance, the surgical tool could be equipped with an accelerometer, or the data processor could additionally use values of current used by the motors of the motorized joints etc...

The storage unit 104 stores, at least, a surgical plan which is renewed at the beginning of each new surgery. The surgical plan defines, at least one initial pose of the surgical tool 121 allowing a user of the system to start treating the region of interest. The surgical plan also defines the relative pose of the region of interest with respect to the anatomical structure. Advantageously, the surgical plan comprises all the successive steps to be performed by the user of the system to treat said region of interest. The surgical plan is thus different for each surgery, and for each patient and can also be modified during an ongoing surgery. Additionally, the surgical plan can also be adapted to the preferences of the user of the system. Optionally, the surgical plan, can define several initial poses of the surgical tool, each initial pose corresponding to the beginning of a particular step of the surgery. For instance, as detailed below, if the surgery consists in drilling one tunnel in a tibial bone and one tunnel in a femoral bone to prepare said bones to receive a ligament graft, the surgical plan can comprise two initial poses permitting, respectively, to drill the tibial tunnel and the femoral tunnel.

The system 100 can be associated with a tracking system configured to determine the relative poses of the surgical tool 121 with respect to the anatomical structure and with respect to the region of interest to be treated and to send a corresponding information to the data processor.

To determine the pose of the surgical tool 121 with respect to the region of interest, the tracking system can for instance be configured to first determine the relative pose of the surgical tool with respect to the anatomical structure. Then, as the pose of the region of interest with respect to the anatomical structure is known, the tracking system is configured to determine the relative pose of the surgical tool with respect to the region of interest, based on the determined pose of the surgical tool with respect to the anatomical structure and on the known pose of the region of interest with respect to the anatomical structure. Alternatively, the tracking system can be configured to determine the relative pose of the surgical tool with respect to the anatomical structure and to send a corresponding information to the data processor, the data processor then being configured to determine the pose of the surgical tool with respect to the region of interest.

The tracking system can be an optical tracking system, thus comprising optical trackers and at least one detecting device, for instance a stereoscopic camera, configured to determine the current poses of the optical trackers and, to compute, consequently, the respective poses of the tracked objects, such as the region of interest and/or the surgical tool. Alternatively, the tracking system could be an electromagnetic tracking system comprising at least two electromagnetic transducers, among which at least one electromagnetic transmitter adapted to emit at least one electromagnetic field, and at least one electromagnetic receiver adapted to receive and measure the emitted electromagnetic field. The tracking system is then configured to determine the relative poses of the electromagnetic transducers with respect to each other and, consequently, to compute the relative poses of the objects to which they are attached, here formed as the surgical tool and the region of interest, based on the measurements of the electromagnetic field. Obviously, these are merely examples of tracking system and such a tracking system could comprise other technologies, such as accelerometers, or inertial sensors, or a combination of the above-cited technologies without departing from the scope of the invention. The tracking system could also be part of the surgical system within the scope of the invention.

According to the invention, the surgical system is operable according to, at least, three distinct modes schematically illustrated by the diagrams shown on figures 2 to 4.

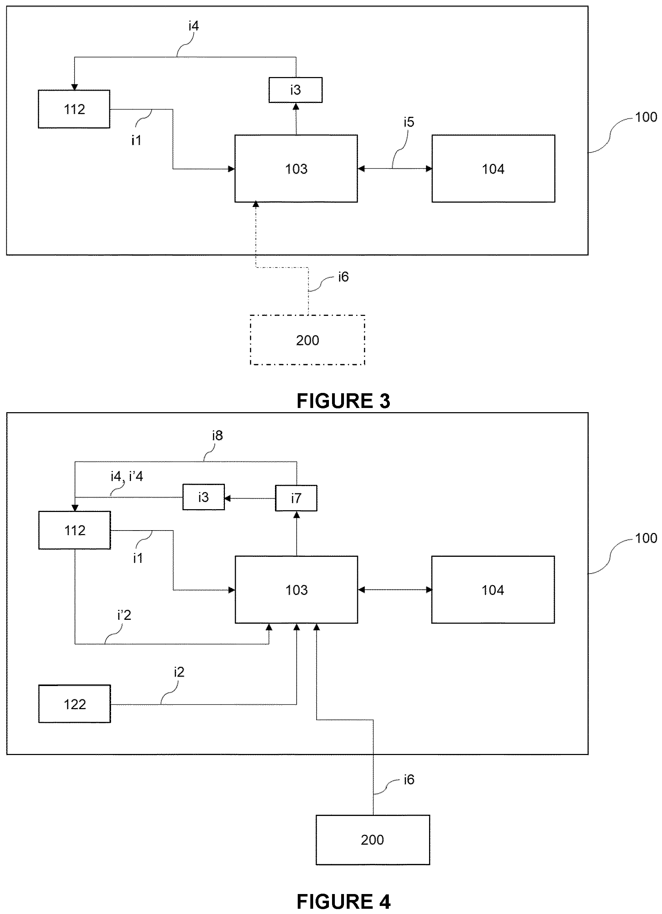

Figure 2 illustrates a diagram representing a hand-guiding mode of the surgical system, figure 3 illustrates a diagram representing a pre-alignment mode of the surgical system 100, and figure 4 illustrated a diagram representing an interactive mode of the surgical system 100.

When the hand-guiding mode is enabled, the robotic arm 110, and consequently the surgical tool coupled to this robotic arm, is displaced, at least partially, based on user commands received through the control device 122 described above.

Especially, the data processor 103 is configured to, as long as the hand-guiding mode is enabled:
- determine the current dynamic condition of the robotic arm 110. A described above, the current dynamic condition of the robotic arm 110 can for instance be determined based, at least, on the information i1 received from the encoders of the motorized joints 112,
- receive at least one user command i2 from the control device 122,
- determine, based on the received user command(s) i2 and on the determined current dynamic condition of the robotic arm 110, an intended displacement of the surgical tool 121,
- determine at least one optimal trajectory i3 of the robotic arm 110 permitting to obtain the determined intended displacement of the surgical tool 121, while satisfying at least one mathematical function which defines authorized trajectories of the robotic arm 110,
- compute and send at least one instruction(s) i4 to at least one of the motorized joint(s) 112 of the robotic arm 110, the execution of the at least one instruction(s) resulting in a displacement of the robotic arm 110 according to the optimal trajectory i3.

According to the invention, when several optimal trajectories of the robotic arm 110 are determined, the data processor 103 is configured to select one of these optimal trajectories so that the displacement of the robotic arm 110 implies the smallest number of movements of the motorized joint(s) 112 of the first part possible 113. A "trajectory of the robotic arm" is defined as successive movements of the motorized joints of this robotic arm. By "select one of the optimal trajectories", we here mean that the data processor 103 is configured to choose one optimal trajectory, among the ones determined as such, for which the corresponding instructions are going to be computed.

By "intended displacement of the surgical tool", we here refer to a displacement the user of the system wants to apply to said surgical tool. As evoked above, when the hand-guiding mode is enabled, the user can transmit his/her intention concerning the displacement of the surgical tool through the control device.

For instance, the at least one mathematical function can be stored in the storage unit 104. This mathematical function is defined as a function which, when satisfied, ensures that the corresponding trajectory of the robotic arm 110 avoids, at least the following issues: singularities, collisions between the robotic arm 110 and its surroundings, collisions between the surgical tool and the robotic arm and collisions between the different segments of the robotic arm. Those issues can be avoided by satisfying several distinct mathematical functions, each of them permitting to avoid at least one of said issues. Alternatively, these distinct mathematical functions could be merged into one unique mathematical function. The mathematical function can also comprise parameters selected by the user of the system, such as preferred trajectories of some motorized joints for instance.

These mathematical function(s) are well known by the skilled man of the art and can for instance comprise the Gilbert Johnson Keerti function, an analytic method of collision resolution and/or a sequential method of collision resolution etc...

Advantageously, the data processor 103 can be configured to detect and/or measure a force and/or torque i'2 applied on at least one of the motorized joints 112 of the second part 114 of the robotic arm 110. For instance, each motorized joint 112 can comprise one forces and/or torques sensor configured to measure de forces and/or torques applied to the concerned motorized joint and to send a corresponding information to the data processor. Alternatively, each motorized joint can comprise two encoders respectively arranged before and after a reductor of the concerned motorized joint, the data processor being configured to receive values measured by said encoders and to compare them in order to determine the forces and/or torques applied to the corresponding motorized joint. According to another alternative, the data processor 103 can receive an information related to the current used by the motor of the concerned motorized joint and to determine, based on this information, on the command sent to the motor and on known parameters of said motor, the force and/or torque applied to the motorized joint.

Thus, the data processor 103 can be further configured to, as long as the hand-guiding mode is enabled:
- determine, based on the detected and/or measured force and/or torque i'2 applied on the concerned motorized joint(s), a configuration of the concerned motorized joint(s) intended by the user,
- select the optimal trajectory i3 of the robotic arm so that the displacement of the concerned motorized joint(s) matches the intended configuration of said motorized joint(s).

Thus, when the hand-guiding mode is enabled, the system of the present invention permits the user to restrain the number of optimal trajectories between which the data processor must choose. Otherwise said, the user can physically lead, at least partially, the selection of the optimal trajectory.

Figure 3 illustrates another mode referred to as "pre-alignment mode". According to the invention, the data processor 103 is configured to, as long as the pre-alignment mode is enabled:
- determine the current configuration of the robotic arm 110, thanks, for instance, to the information i1 transmitted by the encoders of each motorized joint 112
- determine, based on the surgical plan i5 stored in the storage unit 104 and on the current configuration of the robotic arm, a displacement of the surgical tool needed to reach its initial pose,
- determine at least one optimal trajectory of the robotic arm permitting to obtain the determined displacement of the surgical tool, while satisfying the at least one mathematical function which defines authorized trajectories of the robotic arm,
- compute and send at least one instruction(s) i4 to at least one of the motorized joint(s) 112 of the robotic arm 110, the execution of the at least one instruction(s) i4 resulting in a displacement of the robotic arm 110 according to the optimal trajectory,
wherein when several optimal trajectories of the robotic arm 110 are determined, the data processor 103 is configured to select one of these optimal trajectories so that the displacement of the robotic arm 110 implies the smallest number of displacements of the motorized joint(s) 112 of the first part 113 possible.

The initial pose of the surgical tool 121 can be an absolute pose in a defined reference system, or it can be a relative pose with respect to the region of interest. When the initial pose of the surgical tool is registered as a relative pose, the data processor is configured to receive an information i6, from the tracking system 200, related to the current pose of the surgical tool with respect to the region of interest and to use this information i6 concurrently with the stored initial pose registered with the surgical plan i5 and with the determined current configuration of the robotic arm to determine the displacement needed for the surgical tool to reach said relative initial pose. It is understood that as long as the pre-alignment mode is enabled, the user cannot interfere in the selection of the optimal trajectory, nor in the determination of the displacement of the surgical tool.

Additionally, the surgical system 100 can also be operated according to an interactive mode. Figure 4 illustrates, schematically, this interactive mode. According to the invention, the data processor 103 is configured to, as long as the interactive mode is enabled:
- determine the current dynamic condition of the robotic arm, based, at least, on the information i1, for instance, received from the encoders of the motorized joints 112 as described above,
- receive at least one user command i2 from the control device 122,
- determine, based on the received user command(s) i2 and on the current dynamic condition of the robotic arm, the intended displacement of the surgical tool,
- receive the information i6 related to the determined relative pose of the surgical tool with respect to the region of interest, from the tracking system 200,
- determine a predictive pose i7 of the surgical tool with respect to the anatomical structure based on the determined displacement of the surgical tool and on the received information i6 related to the determined relative pose of the surgical tool with respect to the region of interest.

Then, if the predictive pose i7 of the surgical tool with respect to the anatomical structure matches a pose of the region of interest as stored in the surgical plan in the storage unit 104, the data processor 103 is configured to:
- determine at least one optimal trajectory i3 of the robotic arm permitting to obtain the determined displacement of the surgical tool, while satisfying at least one mathematical function which defines authorized trajectories of the robotic arm,
- compute and send at least one instruction(s) i4 to at least one of the motorized joint(s) of the robotic arm, the execution of the at least one instruction(s) resulting in a displacement of the robotic arm according to the optimal trajectory.

If the predictive pose i7 of the surgical tool with respect to the anatomical structure does not match any pose of the region of interest as stored in the surgical plan in the storage unit 104, the data processor 103 can then be configured to send an instruction i8 to brake the motorized joints 112 as a safety measure. Alternatively, the data processor 103 can be configured to adapt the user command for the surgical tool to remain within the region of interest once that the instruction(s) i'4 are executed.

The interactive mode is a mode wherein the user of the system can transmit his/her intention of displacement of the surgical tool, permitting to treat the region of interest, but limited with safety parameter, especially, it is understood from what have been described that the surgical system is configured to comply with the intention of the user only if such intention is coherent with the stored surgical plan.

Optionally, the motorized joints of the first part of the robotic arm can be locked as long as the interactive mode is enabled.

Optionally, the surgical tool can be deactivated as long as one of the pre-alignment mode or the hand-guiding mode is enabled.

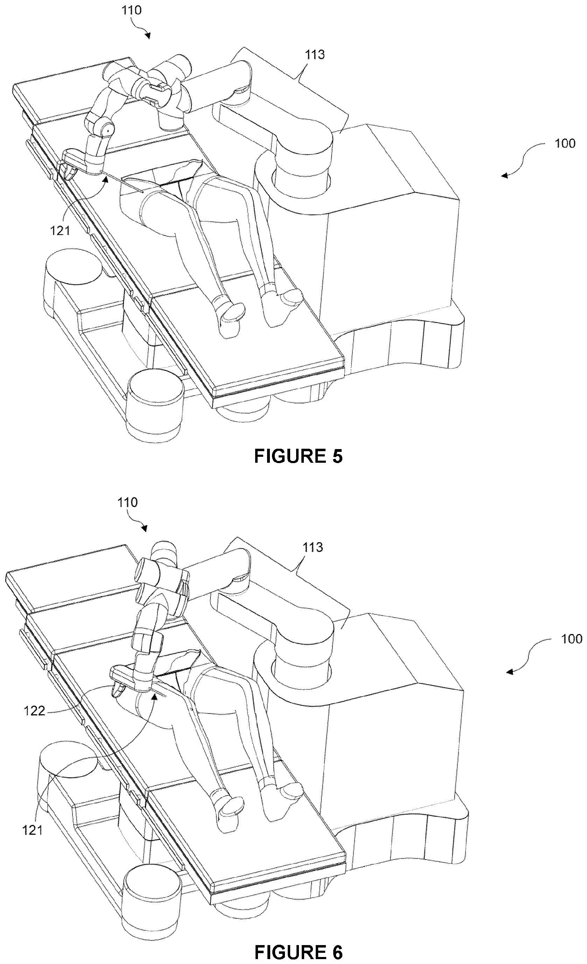

With reference to figures 5 to 8 we are now going to describe the first steps to be carried to perform a knee ligamentoplasty with the surgical system 100 of the invention. As well known in the art, a knee ligamentoplasty must be begun by drilling tunnels adapted to receive the future new ligament - also referred to as a "graft". For replacing an anterior cruciate ligament as illustrated on these figures, at least two tunnels must be drilled, respectively, in the femur and in the tibia. As schematically illustrated on figures 5 to 8, to perform such a surgery the surgical tool 121 can for instance be a drill. A surgical plan for this kind of surgery thus typically comprises, at least, the respective positions of the tunnels to be drilled with respect to the concerned bones, which form the region of interest to be treated and, consequently, the respective poses of the drill forming the trajectory to drill said tunnels.

Figure 5 illustrates a first step wherein the surgical system 100 is operated according to the pre-alignment mode described above with reference to figure 3. As detailed above, the robotic arm 110 is thus configured to position, automatically, based on the stored surgical plan and the information given by the tracking system related to the relative pose of the surgical tool with respect to the region of interest, the surgical tool 121 according to an initial pose registered in the surgical plan and permitting the user to start drilling the femoral tunnel. As previously described, this positioning is advantageously realized according to the selected optimal trajectory of the robotic arm 110. For safety reasons, the surgical system of the invention comprises an activation device, such as a button or a pedal for instance, which must be continuously set off by the user of the system to activate the pre-alignment mode.

Once the surgical drill positioned, the surgical system 100 is switched to be operated according to the interactive mode, for instance described above with reference to figure 4, wherein the user can transmit his/her commands through the control device 122 in order to drill the femoral tunnel, as illustrated on figure 6. According to the example shown, the motorized joints 112 of the first part 113 are locked in position as long as the interactive mode is enabled. As evoked previously, as a tracking system is used, the data processor is configured to ensure that the commands of the user comply with the surgical plan so as to ensure that the drilled tunnel is drilled where planned, i.e. at the location of the region of interest.

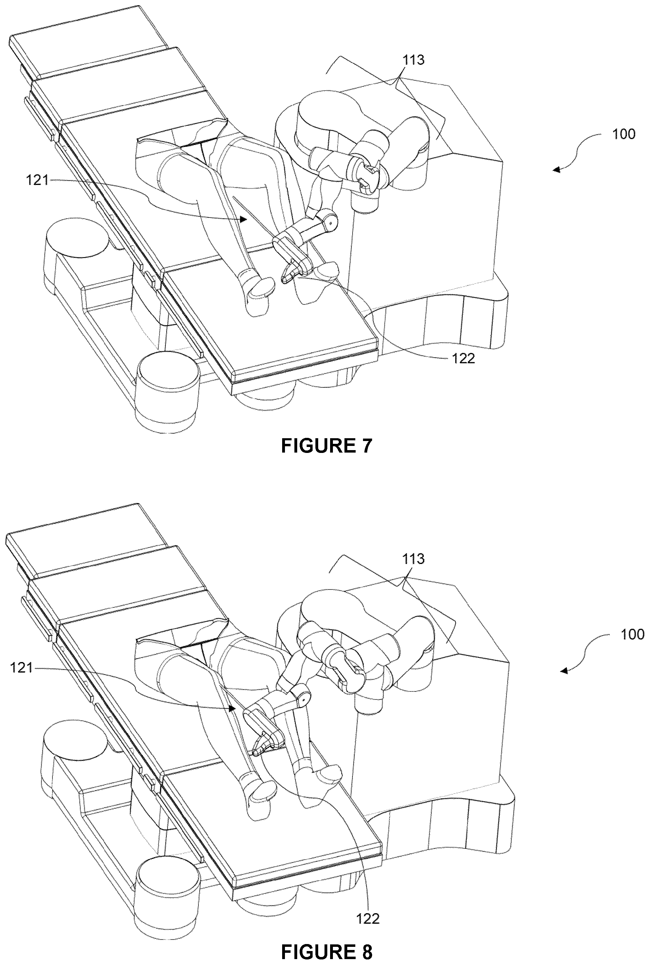

As partially illustrated on figure 7, the user can then pull the drill out of the patient and switch the surgical system back to its pre-alignment mode so that the surgical drill can be positioned, automatically according to the selected optimal trajectory, in the next initial pose, i.e. the stored pose from which the user can start drilling the tibial tunnel. As can be seen from figure 6 to 7, when the pre-alignment is enabled, the motorized joint 112 of the first part 113 are unlocked.

Optionally, the user can switch to the hand-guiding mode, as described with reference to figure 2 after having pulled the drill out of the patient to bring said tool closer to the next initial pose and only then switch to the pre-alignment mode.

Again, once that the surgical drill is positioned, the surgical system 100 can be switched to be operated according to the interactive mode wherein the user can transmit his/her commands through the control device 122 in order to drill the tibial tunnel, as illustrated on figure 8. Again, according to the example shown, the motorized joints 112 of the first part 113 can be locked in position as long as the interactive mode is enabled. Advantageously, as a tracking system is used, the data processor is further configured to ensure that the commands of the user comply with the surgical plan so as to ensure that the drilled tunnel is drilled where planned. The user of the system can thus use the created tunnels to position correctly the new ligament.

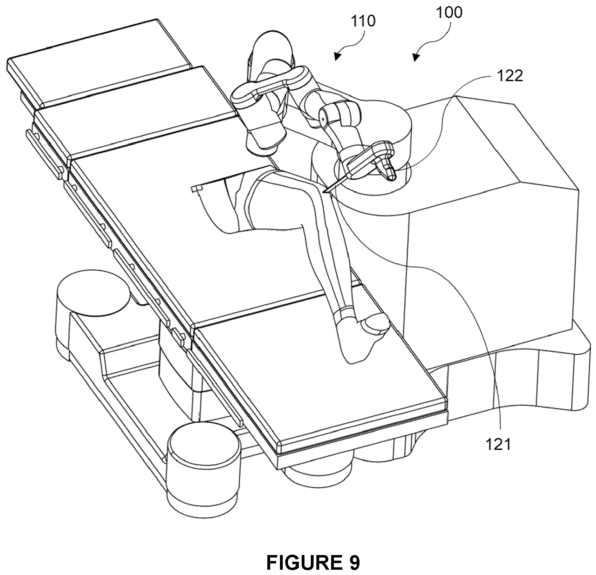

Now referring to figures 9 to 14 the first steps needed to be carried perform a total knee arthroplasty with the surgical system 100 of the invention are illustrated. Generally speaking, a total knee arthroplasty consists in implanting a knee prosthesis to help a patient recover motility of his/her knee, for instance when cartilaginous tissues have been eroded. As well known in the art, a total knee arthroplasty must be begun by machining the femur and the tibia, and especially a distal end of the femur and a proximal end of the tibia, so that the knee prosthesis can be implanted on these bones. As illustrated, to perform such a surgery the surgical tool 121 can for instance be an oscillating saw permitting to realize planar cuts. A surgical plan for this kind of surgery thus typically comprises, at least, the respective positions of six cutting planes, one of which permitting to prepare the tibia to receive a tibial component of the knee prosthesis and five of which permitting to prepare the femur to receive a femoral component of said knee prosthesis. Those cutting planes thus form the region of interest to be treated and, consequently, the respective poses of the oscillating saw forming the trajectory to machine said bones.

Figure 9 illustrates the surgical system 100 operated according to the pre-alignment mode described above with reference to figure 3. As detailed above, the robotic arm 110 is thus configured to position, automatically, based on the stored surgical plan and the information given by the tracking system related to the relative pose of the surgical tool with respect to the region of interest, the surgical tool 121 according to an initial pose registered in the surgical plan and permitting the user to start machining the tibial cut. As previously described, this positioning is advantageously realized according to the selected optimal trajectory of the robotic arm 110. For safety reasons, the surgical system of the invention comprises an activation device, such as a button or a pedal for instance, which must be continuously set off by the user of the system to activate the pre-alignment mode.

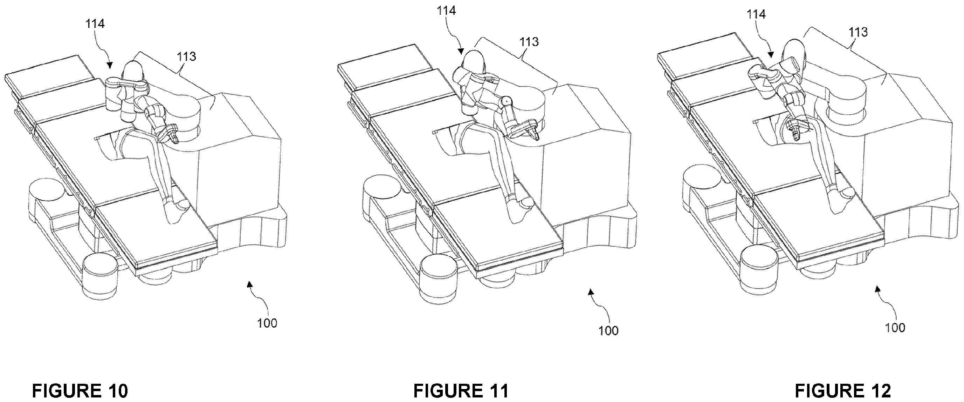

Once the oscillating saw positioned, the surgical system 100 is switched to be operated according to the interactive mode, for instance described above with reference to figure 4, wherein the user can transmit his/her commands through the control device 122 in order to cut the tibial bone, as illustrated on figures 10 to 12. According to the example shown, the motorized joints of the first part 113 are locked in position as long as the interactive mode is enabled. Thus, this first part 113 must be in a position permitting the surgical tool 121 to reach the entire surface of the tibia to be cut only by moving the motorized joints of the second part 114. As evoked previously, as a tracking system is used, the data processor is configured to ensure that the commands of the user comply with the surgical plan so as to ensure that the cut is only performed where planned.

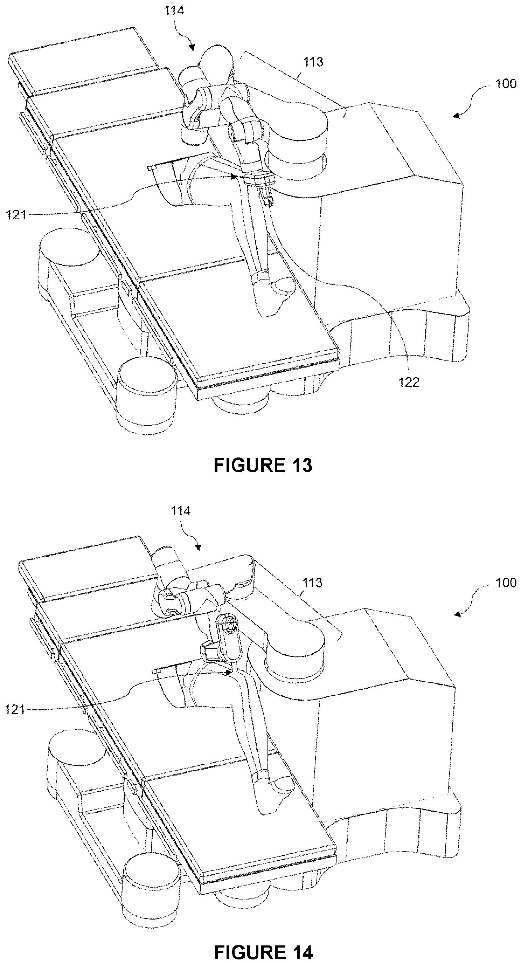

Once that the tibial cut is completed, the user of the system can start the five femoral cuts, only the three first femoral cuts being illustrated, respectively, on figures 13, 14 and 15. To permit the alignment of the surgical tool 121, especially the oscillating saw according to the particular example described, the user can switch the surgical system 100 from the interactive mode to the pre-alignment mode, as described with reference to figure 3. As illustrated, the configuration of the first part 113 can be maintained between the tibial cut and the first femoral cut. Thus, only the second part 114 is displaced to align the surgical tool 121 with the plane of the first femoral cut. The user can then switch the surgical system back to the interactive mode to realize said first femoral cut thanks to the control device 122.

Once that the first femoral cut is completed, the user of the system can start the second femoral cut for instance illustrated on figure 14. Again, to permit the alignment of the surgical tool 121, especially the oscillating saw according to the particular example described, the user can switch the surgical system 100 from the interactive mode to the pre-alignment mode, as described with reference to figure 3. As illustrated, the configuration of the first part 113 can be maintained between the first and the second femoral cut. Thus, only the second part 114 is displaced to align the surgical tool 121 with the plane of the second femoral cut. The user can then switch the surgical system back to the interactive mode to realize said second femoral cut thanks to the control device.

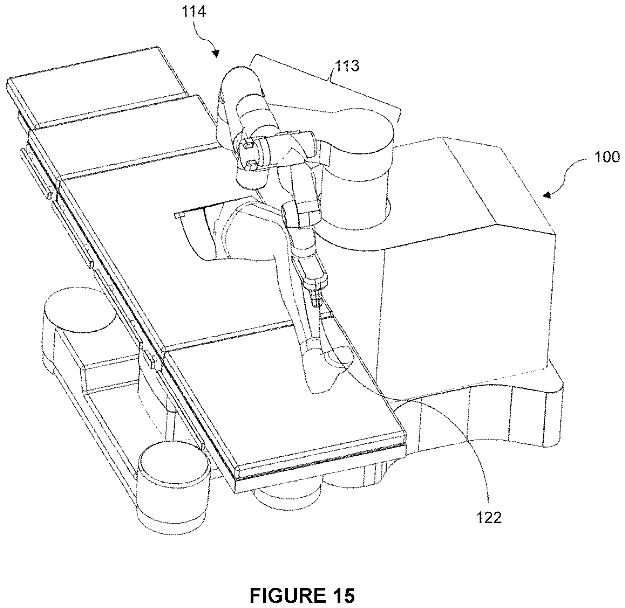

Once that the second femoral cut is completed, the user of the system can start the third femoral cut, for instance illustrated on figure 15. To permit the alignment of the surgical tool, especially the oscillating saw according to the particular example described, the user can switch the surgical system 100 from the interactive mode to the pre-alignment mode, as described with reference to figure 3. As illustrated, the configuration of the first part 113 is modified between the second femoral cut and the third femoral cut, which is allowed, as long as the pre-alignment mode is enabled. Thus, both the first part 113 and the second part 114 are displaced to align the surgical tool with the plane of the third femoral cut. The user can then switch the surgical system back to the interactive mode to realize said third femoral femoral cut thanks to the control device 122.

The described steps can then be repeated as many times as needed to realize all five femoral cuts.

Obviously, those are only examples of applications for which the surgical system of the invention can be used, which does not restrict the invention. The surgical system could be used to perform many other kind of surgeries, and particularly orthopedic surgeries, without departing from the scope of the invention.

## Claims

1. Surgical system (100) configured to treat a region of interest of an anatomical structure, the surgical system (100) comprising
• at least one robotic arm (110) coupled with a surgical tool (121) configured to treat the region of interest, the robotic arm (110) comprising several links (111), two adjacent links (111) being linked to one another by one motorized joint (112), the robotic arm (110) comprising at least a first part (113) with at least three degrees of freedom and a second part (114) with at least six degrees of freedom, an end of the first part (113) forming a base (115) of the second part (114) and the first part (113) comprising a selective compliance assembly robot arm,
• at least one control device (122) configured to receive commands from a user of the system (100), and
• at least one data processor (103),
the surgical system (100) being operable, at least, according to a hand-guiding mode, the data processor (103) being configured to, as long as the hand-guiding mode is enabled:
• determine a current dynamic condition of the robotic arm (110),
• receive at least one user command (i2) from the control device (122),
• determine, based on the received user command(s) (i2) and on the current dynamic condition of the robotic arm (110), an intended displacement of the surgical tool (121),
• determine at least one optimal trajectory (i3) of the robotic arm (110) permitting to obtain the determined intended displacement of the surgical tool (121), while satisfying at least one mathematical function which defines authorized trajectories of the robotic arm (110),
• compute and send at least one instruction(s) (i4) to at least one of the motorized joint(s) (112) of the robotic arm (110), the execution of the at least one instruction(s) (i4) resulting in a displacement of the robotic arm (110) according to the optimal trajectory (i3),
wherein when several optimal trajectories of the robotic arm (110) are determined, the data processor (103) is configured to select one of these optimal trajectories so that the displacement of the robotic arm (110) implies the smallest number of movements of the motorized joint(s) of the first part (113) possible.

2. Surgical system (100) according to claim 1, wherein the control device (122) comprises at least one force and/or torque sensor and wherein the control device (122) is configured to, as long as the hand-guiding mode is enabled:
• measure forces and/or torques applied by the user on the control device (122),
• determine the at least one user command (i2) Fbased on the measured forces and/or torques
• send the user command to the data processor (103).

3. Surgical system (100) accordingto any of claims 1 or 2, wherein the data processor (103) is configured to detect and/or measure a force and/or torque applied on at least one of the motorized joints (112) of the second part (114) of the robotic arm (110), and wherein the data processor (103) is further configured to, as long as one of the hand-guiding mode is enabled:
• determine, based on the detected and/or measured force and/or torque (I'2) applied on the concerned motorized joint(s), a configuration of the concerned motorized joint(s) (112) intended by the user,
• select the optimal trajectory (i3) of the robotic arm (110) so that the displacement of the concerned motorized joint(s) matches the intended configuration of said motorized joint(s).

4. Surgical system (100) according to any of the preceding claims, associated with at least one tracking system (200) configured to determine, at a predefined frequency, relative pose of the surgical tool (121) with respect to the anatomical structure and with respect to the region of interest and to send a corresponding information (i6) to the data processor (103).

5. Surgical system (100) according to the any of the preceding claims, comprising at least one storage unit (104) storing a surgical plan (i5) defining, at least one initial pose of the surgical tool (121) allowing a user of the system (100) to start treating the region of interest, wherein the surgical system (100) is operable according to a pre-alignment mode and wherein the data processor (103) is configured to, as long as the pre-alignment mode is enabled:
• determine a current configuration of the robotic arm (110),
• determine, based on the stored surgical plan (i5) and on the determined current configuration of the robotic arm (110), a displacement of the surgical tool (121) needed to reach its initial pose,
• determine at least one optimal trajectory of the robotic arm (110) permitting to obtain the determined displacement of the surgical tool (121), while satisfying at least one mathematical function which defines authorized trajectories of the robotic arm (110),
• compute and send at least one instruction(s) to at least one of the motorized joint(s) (112) of the robotic arm (110), the execution of the at least one instruction(s) (i4) resulting in a displacement of the robotic arm (110) according to the optimal trajectory,
wherein when several optimal trajectories of the robotic arm (110) are determined, the data processor (103) is configured to select one of these optimal trajectories so that the displacement of the robotic arm (110) implies the smallest number of displacements of the motorized joint(s) of the first part (113) possible.

6. Surgical system (100) according to the preceding claim, wherein the initial pose is registered as an absolute pose in a defined reference system.

7. Surgical system (100) according to claim 4 and 5, wherein the initial pose is registered as relative pose of the surgical tool (121) with respect to the region of interest, and wherein the data processor (103) is configured to, as long as the pre-alignment mode is enabled, determine the displacement of the surgical tool (121) needed to reach its initial pose based on the determined current configuration of the robotic arm (110), on the stored surgical plan and on the information (i6) related to the determined relative pose of the surgical tool (121) with respect to the region of interest.

8. Surgical system (100) according to any of the preceding claims in combination with claim 4, operable in an interactive mode, wherein the data processor (103) is configured to, as long as the interactive mode is enabled:
• determine the current dynamic condition of the robotic arm (110),
• receive at least one user command (i2) from the control device (122),
• determine, based on the received user command(s) (i2) and on the current dynamic condition of the robotic arm (110), the intended displacement of the surgical tool (121),
• receive an information related to the determined relative pose of the surgical tool (121) with respect to the region of interest from the tracking system (200),
• determine a predictive pose (i7) of the surgical tool (121) with respect to the anatomical structure based on the determined intended displacement of the surgical tool (121) and on the received information (i6) related to the determined relative pose of the surgical tool (121) with respect to the region of interest,
wherein if the predictive pose (i7) of the surgical tool (121) with respect to the anatomical structure matches a stored pose of the region of interest, the data processor (103) is further configured to:
• determine at least one optimal trajectory of the robotic arm (110) permitting to obtain the determined intended displacement of the surgical tool (121), while satisfying at least one mathematical function which defines authorized trajectories of the robotic arm (110),
• compute and send at least one instruction(s) to at least one of the motorized joint(s) of the robotic arm (110), the execution of the at least one instruction(s) resulting in a displacement of the robotic arm (110) according to the optimal trajectory.

9. Surgical system (100) according to any of claim 8, wherein, as long as the interactive mode is enabled, the motorized joints (112) of the first part (113) of the robotic arm (110) are locked.

10. Surgical system (100) according to any of the preceding claims, wherein satisfying the at least one mathematical function ensures to avoid one or several of the following:
• Singularities,
• Collisions with objects which are distinct from the robotic arm (110), and which are in the surroundings of the surgical system (100),
• Collisions between distinct link (111)s of the robotic arm (110),
• Collisions between the surgical tool (121) and the robotic arm (110),
• Collisions between the robotic arm (110) and a robot station.

11. Surgical system (100) accordingto any of the preceding claims, wherein the surgical tool (121) is deactivated as long as the hand-guiding mode or the pre-alignment mode is enabled.

12. Surgical system (100) according to any of the preceding claims, comprising exactly one control device (122).

13. Surgical system (100) according to any of the preceding claims, wherein the first part (113) of the robotic arm (110) comprises three motorized joints (112), two of which permittingto displace the base (115) of the second part (114) within a plane and a third one permittingto displace the base of the second part (114) along an axis perpendicular to said plane, and wherein the second part (114) of the robotic arm (110) comprises at least seven motorized joints (112).

14. Surgical system (100) accordingto any of the preceding claims, wherein the surgical tool (121) is a cutting tool or a cutting guide.

15. Method for controlling a surgical system (100) operable in a hand-guiding mode, the surgical system (100) comprising
• at least one robotic arm (110) coupled with a surgical tool (121), the robotic arm (110) comprising several links (111), two adjacent links (111) being linked to one another by one motorized joint (112), the robotic arm (110) comprising at least a first part (113) with at least three degrees of freedom and a second part (114) with at least six degrees of freedom, an end of the first part (113) forming a base of the second part (114) and the first part (113) comprising a selective compliance assembly robot arm,
the method comprising, as long as the hand-guiding mode is enabled, the steps of
• determining a current dynamic condition of the robotic arm (110),
• receiving at least one user command (i2) from the control device (122),
• determining, based on the received user command(s) and on the current dynamic condition of the robotic arm (110), an intended displacement of the surgical tool (121),
• determining at least one optimal trajectory (i3) of the robotic arm (110) permitting to obtain the determined displacement of the surgical tool (121), while satisfying at least one mathematical function which defines authorized trajectories of the robotic arm (110),
• computing and sending at least one instruction(s) (i4) to at least one of the motorized joint(s) of the robotic arm (110), the execution of the at least one instruction(s) (i4) resulting in a displacement of the robotic arm (110) according to the optimal trajectory (i3),
wherein when several optimal trajectories of the robotic arm (110) are determined, the method comprises an additional step of selecting one of these optimal trajectories so that the displacement of the robotic arm (110) implies the smallest number of movements of the motorized joint(s) of the first part (113) possible.
